# EUROPEAN PATENT APPLICATION

(11) **EP 4 717 269 A1**
(43) Date of publication of application: **01.04.2026**
(21) Application number: 24811129.6
(22) Date of filing: 21.05.2024
(51) Int. Cl.: A61K 35/17, A61P 35/00, C12N 5/0783

(54) **COMPOSITION FOR PREVENTING AND/OR AMELIORATING CANCER**

(30) Priority: 22.05.2023 JP 2023084129
(71) Applicant: NATIONAL UNIVERSITY CORPORATION TOKAI NATIONAL HIGHER EDUCATION AND RESEARCH SYSTEM, Nagoya-shi, Aichi 464-8601 (JP); Chubu Pathology Co., Ltd., Gifu 501-6017 (JP)
(72) Inventor: TAKEUCHI, Tamotsu, Gifu City, Gifu 5011193 (JP); SAIGO, Chiemi, Gifu City, Gifu 5011193 (JP); HANAMATSU, Yuki, Gifu City, Gifu 5011193 (JP)
(74) Representative: Hoffmann Eitle
(86) International application number: PCT/JP2024/018759
(87) International publication number: WO 2024/242123

(57) **Abstract**

A primary object of the present disclosure is to provide a composition for preventing and/or ameliorating cancer. The present inventors have found that the above object can be achieved by regulatory T cells that express adiponectin. The present disclosure provides a composition for preventing and/or ameliorating cancer, the composition containing regulatory T cells that express adiponectin.

## Description

### Technical Field

The present disclosure relates to a composition for preventing and/or ameliorating cancer and the like.

### Background Art

Adiponectin is a type of adipocytokine mainly secreted from adipose tissue. Adiponectin is known to have the effects of enhancing insulin sensitivity and promoting fatty acid oxidation. Blood adiponectin concentration is reported to be inversely correlated with BMI (body mass index), visceral fat amount, or the like. That is, the higher the degree of obesity, the lower the blood adiponectin concentration tends to be. Thus, adiponectin is closely associated with obesity.

Recent research suggests a correlation between adiponectin and cancer. For example, Non-patent Literature (NPL) 1 reports that subjects with lower blood adiponectin concentrations potentially have a higher risk of developing cancer and that adiponectin potentially inhibits the development, proliferation, and metastasis of cancer cells through cell cycle arrest and apoptosis.

### Citation List

### Non-patent Literature

NPL 1: Parida et al., "Adiponectin, Obesity, and Cancer: Clash of the Bigwigs in Health and Disease." Int. J. Mol. Sci. 2019, 20, 2519.
NPL 2: Hug C. et al., "T-cadherin is a receptor for hexameric and high-molecular-weight forms of Acrp30/adiponectin." Proc Natl Acad Sci USA. 2004 Jul 13; 101(28): 10308-13.
NPL 3: Toyooka KO et al., "Loss of expression and aberrant methylation of the CDH13 (H-cadherin) gene in breast and lung carcinomas." Cancer Res. 2001 Jun 1; 61(11):4556-60.
NPL 4: Prochazka M. et al., "The nonobese diabetic scid mouse: model for spontaneous thymomagenesis associated with immunodeficiency." Proc Natl Acad Sci USA. 1992 Apr 15; 89(8): 3290-4.
NPL 5: Hanamatsu Y. et al., "Adiponectin-expressing Treg cells in experimental thymic tumor model." Thorac Cancer. 2023 Feb; 14(4):432-433.
NPL 6: Hasegawa M. et al., "Tumor suppressor effect of an antibody on xenotransplanted sarcomatoid mesothelioma cells." Thorac Cancer. 2022 Sep; 13(18): 2566-2573.

### Summary of Invention

### Technical Problem

In view of the above circumstances, the present inventors attempted to ameliorate cancer by using adiponectin. However, as described below, some types of humoral (not enclosed within cells) recombinant adiponectin hardly induced cancer cell death.

Accordingly, the present inventors primarily aimed to provide a composition for preventing and/or ameliorating cancer using adiponectin.

### Solution to Problem

The present inventors found that the above problem can be solved by a composition for preventing and/or ameliorating cancer comprising regulatory T cells that express adiponectin. Based on this finding and further improvements, the present disclosure has been accomplished.

The present disclosure, for example, includes the subject matter listed below.

### Item 1.

A composition for preventing and/or ameliorating cancer, the composition comprising regulatory T cells that express adiponectin.

### Item 2.

The composition according to Item 1, wherein the regulatory T cells interact with cancer cells to exhibit a cell-in-cell phenomenon.

### Item 3.

The composition according to Item 1 or 2, wherein the regulatory T cells express HMW adiponectin.

### Item 4.

The composition according to any one of Items 1 to 3, wherein the cancer has a low adiponectin receptor expression level.

### Item 5.

The composition according to Item 4, wherein the adiponectin receptor is T-cadherin.

### Item 6.

The composition according to any one of Items 1 to 5, wherein the cancer is at least one selected from the group consisting of breast cancer, mesothelioma, bladder cancer, and peritoneally disseminated cancer.

### Item 7.

The composition according to any one of Items 1 to 5, wherein the cancer is breast cancer and/or mesothelioma.

### Item 8.

The composition according to any one of Items 1 to 5, wherein the cancer is triple-negative breast cancer.

### Item 9.

The composition according to any one of Items 1 to 8, which is a pharmaceutical composition.

### Item 10.

A method for producing regulatory T cells that are capable of proliferating in a culture system in the absence of thymic stromal cells and that express adiponectin,
the method comprising the steps of
   (A) co-culturing regulatory T cells that express adiponectin with thymic stromal cells; and
   (B) co-culturing the regulatory T cells that express adiponectin with the thymic stromal cells under conditions in
which the ratio of the thymic stromal cells to the regulatory T cells at the start of co-culture is lower than that at the start of the previous co-culture.

### Item 11.

A method for treating cancer, comprising administering to a subject in need thereof regulatory T cells that express a therapeutically effective amount of adiponectin.

### Item 12.

Use of regulatory T cells in the production of a composition for preventing and/or ameliorating cancer, the regulatory T cells expressing adiponectin.

### Item 13.

A composition for use in the treatment of cancer, the composition comprising regulatory T cells that express adiponectin.

### Advantageous Effects of Invention

The technique of the present disclosure can provide a composition for preventing and/or ameliorating cancer using adiponectin, a method for producing regulatory T cells that express adiponectin, and the like.

### Brief Description of Drawings

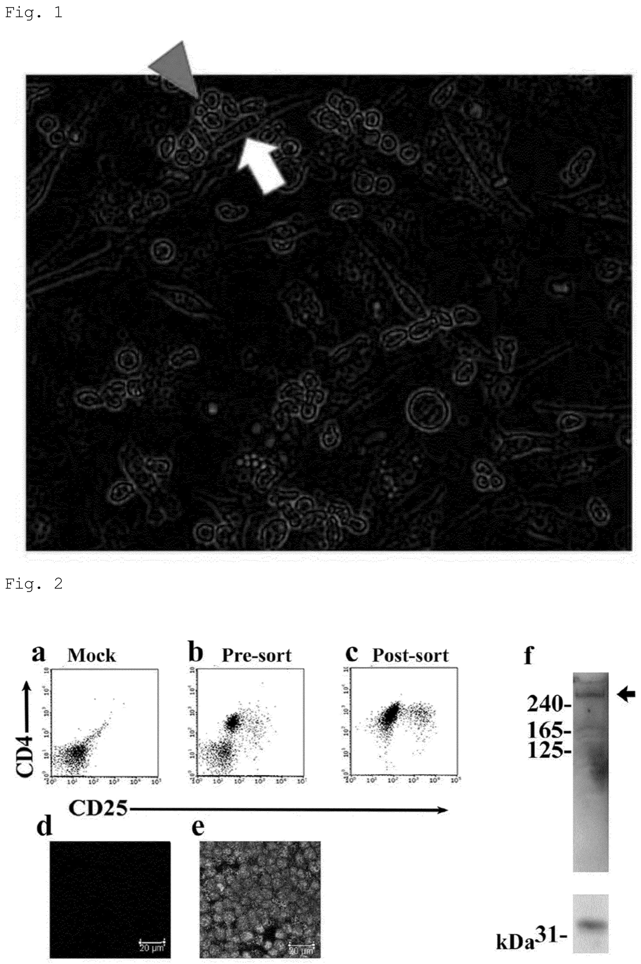

Fig. 1 is a microscopic photograph of cells cultured in Test Example 1-1. In Fig. 1, circular cells marked by the arrowhead are T cells, and a spindle-shaped cell marked by the arrow is a thymic stromal cell.
In Fig. 2, Figs. 2a to 2c show the results of Test Example 1-2. Fig. 2a shows the results of a control that was not incubated with the antibody. Fig. 2b shows the results after incubation with the antibody but before sorting (pre-sort), whereas Fig. 2c shows the results after sorting (post-sort). Figs. 2d and 2e show the results of Test Example 1-3. Fig. 2d shows the results of nuclear staining with DAPI. Fig. 2e shows the results of staining with DAPI (blue), fluorescently labeled rabbit anti-adiponectin antibody (green), and fluorescently labeled mouse anti-FOXP3 antibody (red). In Figs. 2d and 2e, the bars indicate a length of 20 µm. Fig. 2f shows the results of Test Example 1-4. The numerical values shown on the left side of the lane indicate the molecular weight (kDa), and the arrow indicates that HMW adiponectin was expressed.

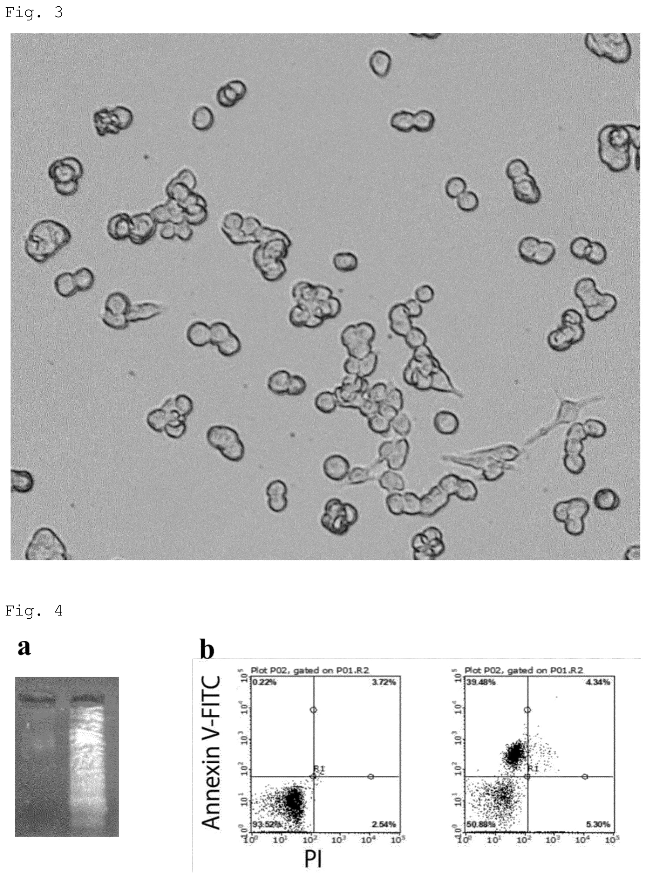

Fig. 3 is a microscopic photograph showing adiponectin-expressing Treg cells capable of autonomous proliferation even in an environment lacking thymic stromal cells, the Treg cells being obtained in Test Example 2-1.
Fig. 4 shows the results of Test Example 2-2. Fig. 4a shows the results of apoptosis detection using chromatin DNA fragmentation into nucleosome units (185 bp) as an indicator, the detection from adiponectin-expressing Treg cells capable of autonomous proliferation being shown in the left lane and the detection from Treg cells capable of proliferating only in a co-culture system with thymic stromal cells being shown in the right lane. Fig. 4b shows the results of apoptosis detection using FITC-labeled annexin V and propidium iodide (PI), the detection from adiponectin-expressing Treg cells capable of autonomous proliferation being shown in the left figure and the detection from Treg cells that proliferate only in a co-culture system with thymic stromal cells being shown in the right figure. Among the four regions in each graph, the plots present in the lower-left region represent live cells, those present in the lower-right region represent early apoptotic cells, and those present in the upper-right region represent late apoptotic cells. Plots present in the upper-left region represent necrotic cells.

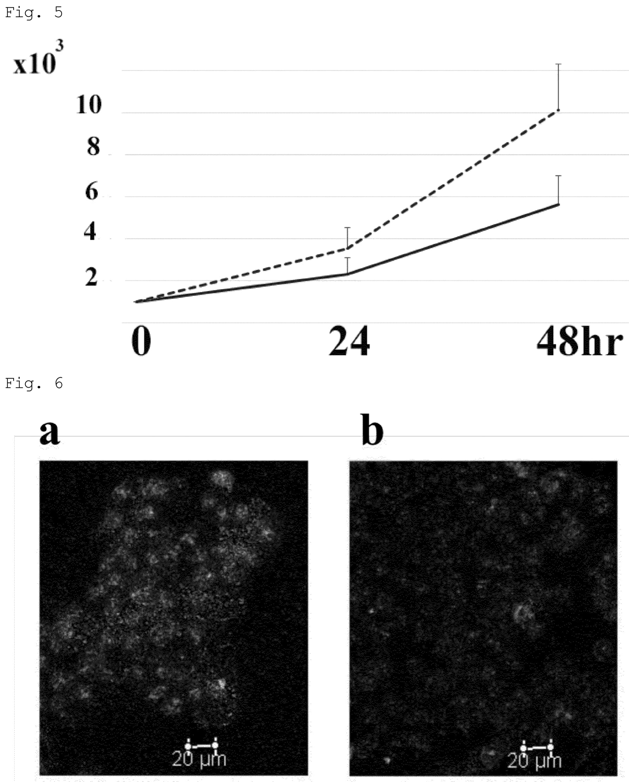

Fig. 5 shows the results of Test Example 2-3. The dashed line represents the culture proliferation results of adiponectin-expressing Treg cells capable of autonomous proliferation, whereas the solid line represents proliferation results of Treg cells capable of proliferating only in a co-culture system with thymic stromal cells. In the graph, the ordinate indicates the number of Treg cells, whereas the abscissa indicates elapsed time from the start of culture. The error bar indicates the standard deviation.
Fig. 6 shows the results of Test Example 2-4. Fig. 6a shows the results of adiponectin-expressing Treg cells capable of autonomous proliferation, which were obtained in Test Example 2-1, whereas Fig. 6b shows the results of Treg cells capable of proliferating only in a co-culture system with thymic stromal cells, which were obtained in Test Example 1-1. The bar shows a length of 20 µm. The expression of adiponectin (indicated by green fluorescence) is greater in adiponectin-expressing Treg cells capable of autonomous proliferation than in Treg cells capable of proliferating only in a co-culture system.

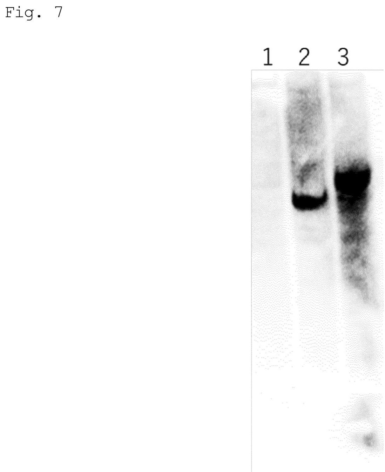

Fig. 7 shows the results of Test Example 2-5. Lane 1 shows the control, Lane 2 shows adiponectin-expressing Treg cells capable of autonomous proliferation obtained in Test Example 2-1, and Lane 3 shows adiponectin-expressing Treg cells sorted from the co-culture system. In Lane 2, uniform production of high-molecular-weight (HMW) adiponectin is observed.

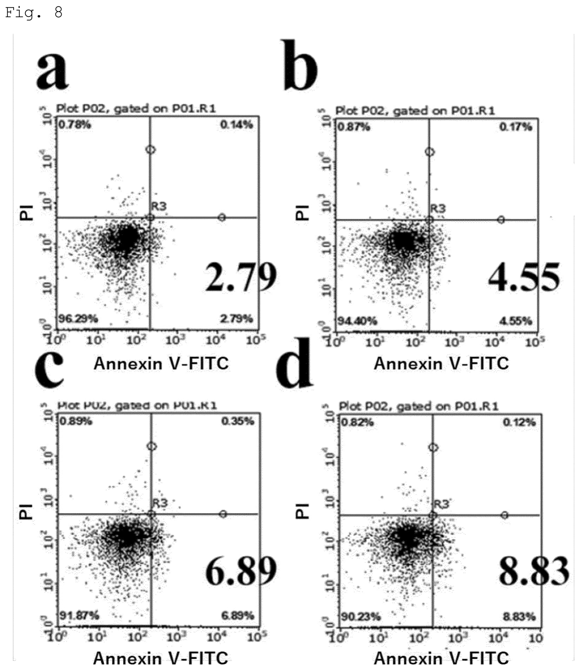

Fig. 8 shows the results of Test Example 3-2. Fig. 8a shows the results at an adiponectin concentration of 0 µg/mL (control), Fig. 8b shows those at 0.05 µg/mL, Fig. 8c shows those at 0.5 µg/mL, and Fig. 8d shows those at 5 µg/mL. The large number shown in each figure indicates the percentage (%) of cells that had undergone apoptosis among the evaluated cells.

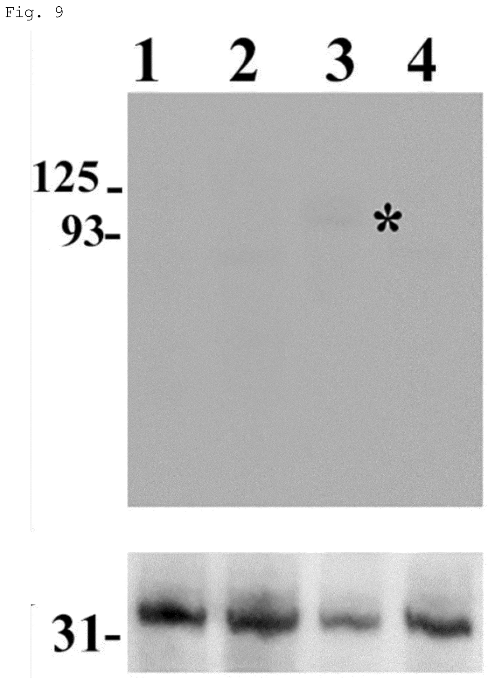

Fig. 9 shows the results of Test Example 4. Lane 1 shows lobular carcinoma cell line MDA-MB-330, lane 2 shows triple-negative breast cancer cell line MDA-MB-231, lane 3 shows triple-negative breast cancer cell line MDA-MB-157, and lane 4 shows luminal A breast cancer cell line MCF-7. The asterisk in lane 3 marks a very faint band of T-cadherin at 105 kDa, while the band around 31 kDa in each lane represents GAPDH (as a loading control).

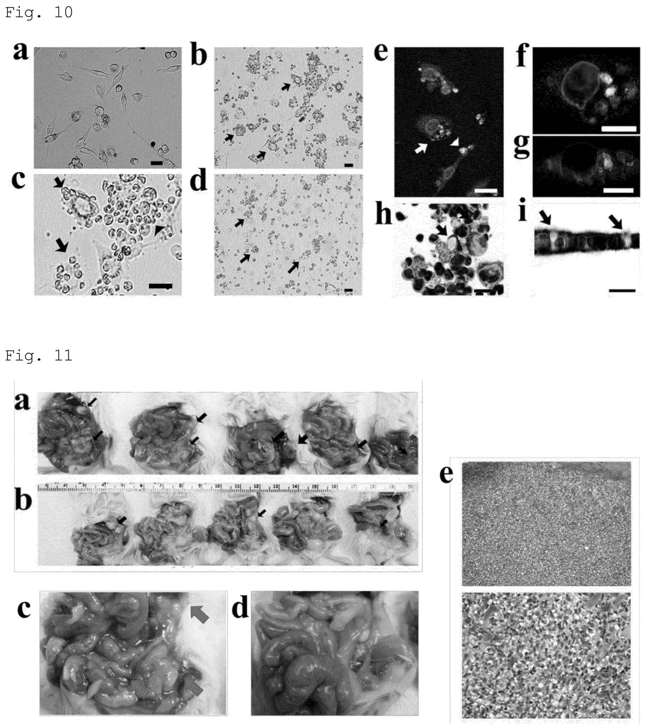

In Fig. 10, Figs. 10a to 10d show the results of Test Example 5-2. Fig. 10a shows MDA-MB-157 cells before co-culture. Figs. 10b and 10c present images from different fields of view taken 16 hours after the start of co-culture. Fig. 10d shows an image captured 64 hours after the start of co-culture. Figs. 10e to 10g show the results of Test Example 5-3. In Fig. 10e, the arrowhead indicates adhesion of an adiponectin-expressing Treg cell to an MDA-MB-157 cell, while the arrow indicates cytoplasmic fusion between an adiponectin-expressing Treg cell and an MDA-MB-157 cell. Fig. 10f shows an intracellular image in the horizontal direction, whereas Fig. 10g shows an intracellular image in the vertical direction of the same subject as in Fig. 10f. Figs. 10h and 10i show the results of Test Example 5-4. The arrow indicates an adiponectin-expressing Treg cell being engulfed by an MDA-MB-231 cell.
Fig. 11 shows the results of Test Example 6. Fig. 11a shows the results of the control group. Fig. 11b shows the results of the group into which adiponectin-expressing Treg cells were injected. In Figs. 11a and 11b, the arrows indicate intraperitoneal mesothelioma masses. Fig. 11c shows a representative case from the control group. Fig. 11d shows a representative case from the group into which adiponectin-expressing Treg cells were injected. In Fig. 11c, the arrow indicates a mesothelioma nodule. Fig. 11e shows histological images of the mesothelioma nodule excised from the control group. The upper photo in Fig. 11e is an image of a HE-stained specimen of the mesothelioma nodule in the control group, whereas the lower photo is an enlarged view of the HE-stained specimen. As shown in Fig. 11e, vigorous invasive proliferation into the striated muscle tissue around the mesothelioma nodule was observed. Such mesothelioma nodules that exhibit vigorous proliferative capacity were observed in the control group shown in Figs. 11a and 11c, whereas such mesothelioma nodules were scarcely detected in the group into which adiponectin-expressing Treg cells were injected, as shown in Figs. 11b and 11d.

### Description of Embodiments

Each embodiment included in the present disclosure is described below in further detail. The present disclosure preferably includes, but is not limited to, a composition for preventing and/or ameliorating cancer, the composition comprising regulatory T cells that express adiponectin; and a method for producing regulatory T cells that express adiponectin. However, the present disclosure is not limited to these, but encompasses everything that is disclosed in the present disclosure and that could be recognized by a person skilled in the art.

The composition included in the present disclosure may be referred to below as "the composition of the present disclosure." The regulatory T cells that express adiponectin may be referred to below as "regulatory T cells of the present disclosure" or "Treg cells of the present disclosure."

Regulatory T cells (which may be referred to as "Treg cells" or simply as "Treg" in the present disclosure) are a type of T cells and known to regulate an immune reaction in the immune system and have the function of preventing overreaction, such as autoimmune diseases and allergic reactions. In the present disclosure, "Treg cells" refer to T cells that express FOXP3 protein in the nucleus. Although not particularly limited, Treg cells contained in the composition of the present disclosure are preferably CD4-positive and/or CD25-positive, and more preferably CD4-positive and CD25-positive.

The Treg cells of the present disclosure express adiponectin. Adiponectin is a protein also known as "Acrp30," "apM1," "GBP28," "AdipoQ," and the like. Adiponectin contains a collagen-like domain at the N-terminal side and a globular domain at the C-terminal side. The monomer of adiponectin has a molecular weight of approximately 30 kDa. As described above, adiponectin is mainly secreted from adipocytes; however, adiponectin has also been reported to be secreted from bone marrow, osteoblasts, fetal tissues, muscle cells, cardiomyocytes, salivary gland epithelial cells, and the like.

The Treg cells of the present disclosure are not particularly limited in origin as long as they are Treg cells that express adiponectin. For example, adiponectin-expressing Treg cells derived from living organisms may be selected. Treg cells that express adiponectin may also be obtained by introducing a construct encoding the sequence for expressing adiponectin into Treg cells that do not express adiponectin. Further, Treg cells that express adiponectin may also be obtained by differentiating T cell precursors that express adiponectin into Treg cells.

A more specific method for obtaining Treg cells that express adiponectin comprises, for example, excising a thymic tumor, culturing the tumor cells, and then sorting T cells using CD4 and/or CD25 positivity as an indicator. Thymic tumors obtained from, for example, non-obese diabetic / severe combined immunodeficient (NOD/SCID) mice can be used. The presence of Treg cells in the obtained cells can be confirmed by using FOXP3 expression as an indicator. The obtained cells can be sorted by using FOXP3 positivity as an indicator in order to increase the proportion of Treg cells in the obtained cells. The obtained cells can be sorted using adiponectin expression as an indicator in order to confirm that the cells express adiponectin and to increase the proportion of adiponectin-expressing Treg cells in the obtained cells.

Examples of organisms from which Treg cells can be derived include humans and non-human mammals (such as rats, mice, rabbits, cattle, pigs, dogs, cats, sheep, and monkeys).

In vivo, adiponectin is present in various isoforms, ranging from trimers to high molecular weight (HMW) multimers, each of which has been reported to possess different activities and functions. For example, adiponectin trimers and hexamers are suggested to be involved in the regulation of food intake, whereas high-molecular-weight (HMW) adiponectin (which may be referred to as "HMW adiponectin" in the present disclosure) is primarily involved in insulin sensitivity, hepatic gluconeogenesis, and other metabolic regulation. Adiponectin isoforms are believed to be determined through intracellular processes, such as post-translational modifications, and it is thought that interconversion between isoforms does not occur once they are secreted outside the cell (Non-patent literature (NPL) 1).

In the technique of the present disclosure, the isoform of adiponectin is not particularly limited as long as the effects of the present disclosure are achieved. The isoform may be a monomer, a trimer, a hexamer, or an HMW multimer. However, the isoform is preferably a hexamer or an HMW multimer capable of binding to T-cadherin, and is more preferably an HMW multimer.

In the present disclosure, the term "HMW adiponectin" refers to an adiponectin multimer with a molecular weight of 190 kDa or more. The molecular weight of HMW adiponectin is not particularly limited as long as it is 190 kDa or more. For example, the molecular weight may be 190 to 600 kDa, preferably 200 to 500 kDa, and more preferably 240 to 400 kDa. The upper or lower limit of the molecular weight of HMW adiponectin may be 200, 220, 240, 260, 280, 300, 320, 340, 360, 380, 400, 420, 440, 460, 480, 500, 520, 540, 560, 580, or 600 kDa.

As adiponectin receptors, AdipoR1, AdipoR2, and T-cadherin (also referred to as "H-cadherin" or "cadherin-13") have been identified. Among these receptors, T-cadherin has been reported to be a receptor for both a hexamer and HMW adiponectin (Non-patent Literature (NPL) 2). T-cadherin is expressed on the cell membrane surface and has an adiponectin-binding site in its extracellular domain.

Decreased expression levels of adiponectin receptors in cancer cells have been reported. The decrease in adiponectin receptor expression levels has been suggested to result from abnormal methylation of the promoter region in the adiponectin receptor gene (Non-patent Literature (NPL) 3). Although not limiting, the composition of the present disclosure is suitably used for cancers that exhibit low expression levels of adiponectin receptors, and is especially suitable for cancers in which the expression of T-cadherin is decreased.

In the present disclosure, a "decrease in adiponectin receptor expression level" refers to a reduction in the expression level of normal (i.e., functional) adiponectin receptors in cancer cells, which are the target of the composition of the present disclosure, as compared to that in normal cells derived from the same tissue of origin. Accordingly, even if cancer cells express a considerable amount of abnormal (i.e., loss-of-function) adiponectin receptors, a reduced expression level of normal adiponectin receptors in the target cells compared with that in normal cells is considered to be a decrease in adiponectin receptor expression level. The term "expression level" encompasses both the amount of transcript and the amount of translation product.

The degree of decrease in adiponectin receptor expression may be, for example, 3/4 or 2/3 or less, preferably 1/2, 1/3, or 1/4, more preferably 1/5, 1/6, 1/7, 1/8, 1/9, or 1/10, even more preferably 1/20, 1/25, 1/30, 1/40, 1/50, 1/60, 1/70, 1/80, 1/90, or 1/100, compared to the adiponectin receptor expression in normal cells, and is particularly preferably below the detection limit.

The decrease in adiponectin receptor expression level can be determined by known methods or methods that are easily conceivable from known methods. Examples of the method for determining the decrease in adiponectin receptor expression level include reverse transcription quantitative PCR (RT-qPCR), RNA-seq, western blotting, and immunohistochemical staining.

The Treg cells of the present disclosure preferably interact with cancer cells to exhibit a cell-in-cell phenomenon. The "cell-in-cell phenomenon" refers to a phenomenon in which one cell is engulfed by another cell. That is, it is preferable that the Treg cells of the present disclosure can be engulfed by cancer cells.

The composition of the present disclosure is suitably used for the prevention, amelioration, and treatment of cancer. In the present disclosure, the term "preventing cancer" includes applying the composition to a subject before a cancer diagnosis to prevent the onset of cancer, as well as applying the composition to tissues from which cancer has not been detected in a diagnosed subject to prevent metastasis. In the present disclosure, the terms "ameliorating cancer" and "treating cancer" include applying the composition to a subject diagnosed with cancer to suppress cancer progression, halt its advancement, inhibit the proliferation of cancer cells, kill cancer cells, reduce the number of cancer cells, shrink tumor volume, and prevent cancer metastasis. Further, in the present disclosure, the term "anti-cancer effect" includes effects of preventing, ameliorating, and treating cancer.

In the present disclosure, the term "cancer" includes cancers, neoplasms, and malignant tumors. Examples of cancers include breast cancer, mesothelioma, bladder cancer, peritoneally disseminated cancer, lung cancer, colorectal cancer, stomach cancer, soft tissue sarcoma, osteosarcoma, brain tumors, neuroblastoma, leukemia, lymphoma, liver cancer, kidney cancer, prostate cancer, retinoblastoma, ciliary body tumors, malignant melanoma, basal-cell carcinoma, squamous-cell carcinoma, malignant soft-tissue tumors, chondrosarcoma, renal-cell carcinoma, ovarian cancer, uterine corpus cancer, and cervical cancer.

The cancer to which the composition of the present disclosure is applied is preferably at least one selected from the group consisting of breast cancer, mesothelioma, bladder cancer, and peritoneally disseminated cancer, among the cancers mentioned above, and more preferably breast cancer and/or mesothelioma. "Disseminated cancer" refers to a condition in which cancer cells have metastasized and spread to locations distant from the primary site. "Peritoneally disseminated cancer" refers to a condition in which cancer cells have spread within the peritoneal cavity, metastasizing to organs such as the stomach, small intestine, large intestine, liver, and pancreas, as well as to the peritoneal walls and peritoneal surfaces.

The composition of the present disclosure can be particularly suitably applied to triple-negative breast cancer among breast cancers. "Triple-negative breast cancer" refers to breast cancer that is negative for all of the following: estrogen receptor, progesterone receptor, and HER2 receptor (that is, triple-negative). When the cancer is positive for at least one of the receptors selected from estrogen receptor, progesterone receptor, and HER2 receptor, treatments such as hormone therapy and targeted therapy are effective. However, general hormone therapy and targeted therapy are not effective for triple-negative breast cancer, which is negative for these receptors. Triple-negative breast cancer is presumed to have a high biological malignancy among breast cancers.

As described below, the composition of the present disclosure is believed to improve cancer through a mechanism of action different from that of estrogen receptor, progesterone receptor, and HER2 receptor; and therefore, the composition can be suitably used even for triple-negative breast cancer for which general hormone therapy and targeted therapy are ineffective.

The composition of the present disclosure is not particularly limited and can be, for example, a pharmaceutical composition.

Examples of subjects to which the composition of the present disclosure is applied include humans and non-human mammals (such as rats, mice, rabbits, cattle, pigs, dogs, cats, sheep, and monkeys), with humans being particularly preferred.

The method for applying the composition of the present disclosure is not particularly limited as long as the desired effect is achieved. For example, the composition of the present disclosure may be administered by direct injection into the lesion, or injected or infused intravenously, intra-arterially, intraperitoneally, intramuscularly, subcutaneously, intrapleurally, etc., or may be administered by perfusion with a catheter. Direct injection into the lesion is preferable among such administration methods.

The frequency of application of the composition is not particularly limited. For example, the composition can be applied once or multiple times a day, once or multiple times a week, once or multiple times a month, or once or multiple times a year. Further, the duration of application of the composition of the present disclosure is also not particularly limited as long as the effects of the present disclosure are provided. Further, the dosage of the composition of the present disclosure is also not particularly limited as long as the effects of the present disclosure are provided. The frequency and duration of application, as well as the dosage, may be appropriately adjusted by those skilled in the art according to, for example, the condition of the subject and the course of treatment.

The composition of the present disclosure may be used in combination with other pharmaceutical compositions and/or other therapeutic methods that are applied to subjects having cancer. When the composition of the present disclosure is combined with another pharmaceutical composition and/or another therapeutic method, they may be applied to the subject simultaneously or separately at any time.

The composition of the present disclosure may contain, in addition to the Treg cells of the present disclosure, pharmaceutically acceptable bases, carriers, excipients, diluents, solubilizers, emulsifiers, preservatives, pH adjusters, adjuvants, or other additives, either alone or in a combination of two or more.

Examples of preservatives include parabens, such as methylparaben, ethylparaben, propylparaben, and butylparaben; sodium benzoate, phenoxyethanol, and alkyldiaminoethylglycine hydrochloride. Preservatives can be used alone or in a combination of two or more.

Examples of pH adjusters include citric acid, phosphoric acid, malic acid, pyrophosphoric acid, lactic acid, tartaric acid, glycerophosphoric acid, acetic acid, and nitric acid, as well as chemically acceptable salts of these acids, sodium hydroxide, potassium hydroxide, and the like. pH adjusters can be used alone or in a combination of two or more to adjust the pH of the composition to a range of 4 to 8, preferably 5 to 7.

The method for preparing the composition of the present disclosure is not particularly limited as long as the effects of the present disclosure are provided. For example, the composition of the present disclosure can be prepared in accordance with a method known in this technical field. More specifically, for example, the composition of the present disclosure can be prepared by mixing the Treg cells of the present disclosure and other components with sterile distilled water.

As stated above, decreased expression levels of adiponectin receptors in cancer cells have been reported. Without wishing to be bound by theory, in the technique of the present disclosure, since adiponectin is enclosed within Treg cells, adiponectin is presumed to be internalized into cells through a cell-in-cell phenomenon regardless of the presence or absence of adiponectin receptors, thus exerting anti-cancer effects.

The present disclosure further includes a method for producing regulatory T cells that are capable of proliferating in a culture system in the absence of thymic stromal cells and that express adiponectin. This production method may be referred to below as "the production method of the present disclosure." In the present disclosure, being capable of proliferating in a culture system in the absence of thymic stromal cells may be referred to as "capable of autonomous proliferation" or "autonomously proliferating."

As described below, Non-patent Literature (NPL) 5 reports that isolated regulatory T cells that express adiponectin undergo cell death (apoptosis) in an environment lacking thymic stromal cells. According to the production method of the present disclosure, adiponectin-producing Treg cells capable of proliferating even in an environment lacking thymic stromal cells can be established.

Adiponectin-expressing Treg cells capable of proliferating even in an environment lacking thymic stromal cells are preferable from the standpoint that, unlike Treg cells capable of proliferating only in a co-culture system with thymic stromal cells, a step of separating adiponectin-expressing cells from thymic stromal cells is not required before use (e.g., application to a subject). Adiponectin-expressing Treg cells capable of autonomous proliferation can have excellent characteristics in terms of proliferation efficiency and expressed adiponectin isoform uniformity and are thus preferable from this viewpoint as well.

The production method of the present disclosure comprises the steps of
(A) co-culturing regulatory T cells that express adiponectin with thymic stromal cells; and
(B) co-culturing the regulatory T cells that express adiponectin with the thymic stromal cells under conditions in which the ratio of the thymic stromal cells to the regulatory T cells at the start of co-culture is lower than that at the start of the previous co-culture.

In step (B), the embodiment in which the ratio of the thymic stromal cells to the Treg cells at the start of co-culture compared to that at the start of the previous co-culture is not particularly limited as long as the desired effects are obtained. For example, when the first co-culture is started at an adiponectin-expressing Treg cell/thymic stromal cell ratio of 100:1, the second co-culture may be started at an adiponectin-expressing Treg cell/thymic stromal cell ratio of 1000:1. The thymic stromal cells are adherent to the culture dish, whereas the adiponectin-expressing Treg cells are non-adherent (i.e., suspended) to the culture dish. Accordingly, the thymic stromal cell fraction can be obtained in the state of adhering to the culture dish by aspirating and removing the culture medium from the co-culture system, whereas the adiponectin-expressing Treg cell fraction can be obtained in the state of being suspended in the culture medium by centrifuging the medium. The process from the start of co-culture to the separation of Treg cells from the co-culture system after any desired period of co-culture is counted as one co-culture cycle.

As long as the desired effects are obtained, step (B) may be performed once or multiple times. When performed multiple times, step (B) may be carried out, for example, once to 10 times, and preferably once to 5 times. The upper or lower limit of the range may be 1, 2, 3, 4, 5, 6, 7, 8, 9, or 10 times.

The ratio of thymic stromal cells to Treg cells at the start of each co-culture is not particularly limited as long as it is smaller than the ratio of thymic stromal cells to Treg cells at the start of the previous co-culture. For example, the ratio of adiponectin-expressing Treg cells to thymic stromal cells may be from 1 : 1 to 100000 : 1, preferably 5 : 1 to 10000 : 1, and more preferably 10 : 1 to 1000 : 1. The upper or lower limit of the range of the ratio may be 1, 2, 5, 7, 10, 20, 50, 70, 100, 200, 500, 700, 1000, 2000, 5000, 7000, 10000, 20000, 50000, 70000, or 100000 : 1.

The duration of co-culture in steps (A) and (B) is also not particularly limited and can be, for example, 1 to 30 days, preferably 5 to 10 days, from the start of co-culture. The upper or lower limit of the range of the duration may be 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days, or may be 1, 2, or 3 weeks. Further, the duration of each co-culture cycle may be the same or may differ from one another.

Embodiments of the production method of the present disclosure are described below. However, the production method of the present disclosure is not limited to the following examples. A mixed cell population of Treg cells and thymic stromal cells is cultured in a culture dish (step (A)). The culture medium is aspirated from the culture dish to obtain a thymic stromal cell fraction adhering to the dish. The aspirated culture medium is centrifuged to obtain an adiponectin-expressing Treg cell fraction. Subsequently, co-culture is started at a ratio of isolated adiponectin-expressing Treg cells to thymic stromal cells of 100:1. One week after the start of co-culture, thymic stromal cells and adiponectin-expressing Treg cells are separated by the above method (first co-culture cycle of step (B)). Co-culture is started at a ratio of isolated adiponectin-expressing Treg cells to thymic stromal cells of 10000:1. After one week, the culture medium is collected and centrifuged to obtain adiponectin-expressing Treg cells (second co-culture cycle of step (B)).

The production method of the present disclosure preferably includes, in addition to steps (A) and (B), step (C) of culturing adiponectin-expressing Treg cells that have been separated after co-culture, without mixing thymic stromal cells (i.e., culturing adiponectin-expressing Treg cells alone) to confirm that adiponectin-expressing Treg cells are capable of autonomous proliferation. If autonomous proliferation of adiponectin-expressing Treg cells is not confirmed in step (C), step (B) may be further repeated until autonomous proliferation is confirmed.

In the technology of this disclosure, the method of culturing cells, such as culture medium, culture vessel, culture conditions, duration of culture, and passaging method, is not particularly limited. Known methods or methods that can be easily conceivable from known methods can be used.

The culture medium to be used can be, for example, a conventionally known basal medium supplemented with any optional components that may be added to the medium. Examples of known basal media include Dulbecco's Modified Eagle Medium (DMEM), Ham's F12 medium, DMEM/F12 medium, IMDM, and EMEM. Examples of any optional components that can be added to the culture medium include, but are not limited to, fetal bovine serum (FBS), bovine serum albumin (BSA), transferrin, insulin, ethanolamine, fatty acids, collagen precursors, trace elements, 2-mercaptoethanol, thioglycerol, lipids, amino acids, vitamins such as ascorbic acid, growth factors such as EGF, antibiotics such as penicillin and streptomycin, antioxidants, buffers, and inorganic salts. The optional components that can be added to the culture medium can be used alone or in a combination of two or more. In the present disclosure, the components contained in the medium and their contents are not particularly limited as long as they do not inhibit cell proliferation. The same culture medium composition can be used throughout the culture period, or one medium can be replaced with another having a different composition at any time. The culture medium for culturing Treg cells of the present disclosure is, for example, preferably DMEM medium containing 10% fetal bovine serum (FBS).

The conditions for culturing the cells are not particularly limited as long as cell proliferation is not inhibited. The culture temperature is, for example, about 20 to 45°C, preferably about 30 to 40°C, more preferably about 35 to 40°C, and particularly preferably about 37°C. The CO₂ concentration is also not particularly limited, and can be, for example, about 5%.

Passaging may be performed as appropriate according to the condition of the cells. For example, passaging may be performed when the cells grow and the cell occupancy area ratio (confluency) has reached about 80% or more. Further, the medium can be replaced as appropriate according to the condition of the cells. The medium after the medium replacement may have the same composition as the medium before the replacement or may have a different composition.

In the present specification, the term "comprising" includes not only containing but also essentially consisting of and consisting of. Furthermore, the present disclosure encompasses all possible combinations of the constituent elements described in the present specification.

Various characteristics (properties, structures, functions, etc.) described in the above embodiments of the present disclosure may be combined in any manner to specify the subject matter included in the present disclosure. That is, the present disclosure includes all of the subject matter comprising any combination of the combinable properties described herein.

### Examples

The embodiments of the present disclosure are more specifically explained below with reference to Examples. However, the embodiments of the present disclosure are not limited to the Examples described below.

### Test Example 1. Isolation of Regulatory T Cells That Express Adiponectin

1-1.
A thymic tumor was excised from a 14-week-old non-obese diabetic/severe combined immunodeficient (NOD/SCID) mouse (strain name: NOD.CB17-Prkdc ^{scid}/J, available from Charles River Laboratories). The mouse is a model mouse that is created by introducing Prkdcscid mutation from a SCID mouse into an NOD mouse by crossbreeding and that has the genetic background of the NOD mouse exhibiting low NK cell activity in combination with congenital T and B lymphocyte deficiency of the SCID mouse, which is severe immunodeficiency (Non-patent Literature (NPL) 4).

The excised thymic tumor cell mass was minced into small pieces and then cultured in antibiotic-free DMEM medium supplemented with 10% heat-inactivated fetal bovine serum (FBS). The cell culture was performed using 35-mm culture dishes in a 5% CO₂ incubator at 37°C. Fig. 1 shows a microscopic photograph of the cultured cells. In Fig. 1, the circular cells indicated by an arrowhead are T cells, whereas the spindle-shaped cell indicated by an arrow is a thymic stromal cell.

1-2.
The cells obtained in Test Example 1-1 were subjected to fluorescent immunostaining using anti-CD4 and anti-CD25 antibodies to sort T cells. Specifically, after the cells were incubated with rat anti-mouse CD4 antibody (Sony Corporation) conjugated with phycoerythrin (PE) and rabbit monoclonal anti-mouse CD25 antibody (available from Sino Biological) conjugated with fluorescein isothiocyanate (FITC), the cells were sorted using an SH800 cell sorter (available from Sony Corporation). Fig. 2a shows the results of the control sample that was not incubated with any antibody. Fig. 2b shows the results after incubation with the antibody but before sorting (pre-sort). Fig. 2c shows the results after sorting (post-sort).

1-3.
The CD4- and CD25-positive T cells sorted in Test Example 1-2 were stained with DAPI (4',6-diamidino-2-phenylindole) for nuclear visualization and observed using a confocal laser scanning microscope (Leica). Fig. 2d shows the results.

Further, the T cells were immunostained with DAPI (blue), fluorescently labeled rabbit anti-adiponectin antibody (green) (GeneTex, Inc.), and fluorescently labeled mouse anti-FOXP3 antibody (red) (Proteintech, Inc.), and were observed with a confocal laser scanning microscope (Leica). Fig. 2e shows the results. In Figs. 2d and 2e, the bars represent 20 µm.

In Fig. 2e, the pink indicates the overlap of the nucleus immunostained in blue and FOXP3 immunostained in red. The results revealed that FOXP3 is expressed in the nucleus of the cells sorted in Test Example 1-2, that is, the cells are regulatory T cells (Treg cells). Further, as shown in green in Fig. 2e, the results revealed that adiponectin is expressed in the cytoplasm of the Treg cells. The variation in intensity of green in Fig. 2e indicates that the levels of adiponectin expression in Treg cells are different from cell to cell.

1-4.
Adiponectin was detected by western blotting in the Treg cells sorted in Test Example 1-2.

Specifically, after the cell lysate was subjected to electrophoresis using sodium dodecyl sulfate-polyacrylamide gel (SDS-PAGE), the proteins were transferred onto a polyvinylidene fluoride (PVDF) membrane (Immobilon-P Transfer Membrane; Merck). The membrane was blocked with blocking milk (Snow Brand Megmilk Co., Ltd.) and incubated with a rabbit anti-adiponectin antibody. For immunodetection, horseradish peroxidase (HRP)-labeled anti-rabbit secondary antibody (Cell Signaling Technology) and an ultra-sensitive HRP chemiluminescent substrate (TaKaRa Bio Inc.) were used. The images were obtained using the Invitrogen iBright 1500 gel imaging system (Thermo Fisher Scientific). After stripping antibodies from the membrane, the membrane was incubated with anti-GAPDH antibody (Merck) to evaluate the input. Fig. 2f shows the results. The numbers on the left side of the lane indicate the molecular weight (kDa).

As indicated by the arrow in Fig. 2f, the Treg cells expressed HMW adiponectin with a molecular weight of greater than 240 kDa. It is also confirmed that in addition to HMW adiponectin, adiponectin having various molecular weights was expressed. The band near 31 kDa in Fig. 2f indicates GAPDH (loading control).

### Test Example 2: Establishment of Autonomously Proliferative Adiponectin-expressing Treg Cells

Non-patent literature (NPL) 5 reports that isolated regulatory T cells expressing adiponectin undergo cell death (apoptosis) in an environment lacking thymic stromal cells.

Accordingly, the present inventors attempted to establish adiponectin-expressing Treg cells capable of autonomous proliferation even in an environment lacking thymic stromal cells.

2-1.
The present inventors gradually reduced the proportion of thymic stromal cells in the co-culture system of thymic stromal cells and adiponectin-expressing Treg cells, eventually achieving a state in which no thymic stromal cells were present.

Specifically, first, a mixed cell population of Treg cells obtained in Test Example 1-1 and thymic stromal cells were cultured in a culture dish. As described above, thymic stromal cells are adherent to culture dishes, whereas adiponectin-expressing Treg cells are non-adherent to culture dishes (i.e., suspended). After aspirating the culture medium from the culture dish, the thymic stromal cell fraction adhering to the dish was obtained. The aspirated culture medium was centrifuged to obtain an adiponectin-expressing Treg cell fraction.

Subsequently, co-culture was started at a 100:1 ratio of adiponectin-expressing Treg cells to thymic stromal cells. One week after the start of the co-culture, the thymic stromal cells and the adiponectin-expressing Treg cells were separated by using the method described above, and co-culture was started at a ratio of adiponectin-expressing Treg cells to thymic stromal cells of 10000 : 1. After one week, the culture medium was collected and centrifuged to obtain adiponectin-expressing Treg cells.

The separated adiponectin-expressing Treg cells were cultured in the absence of thymic stromal cells for 1 week. Proliferation of adiponectin-expressing Treg cells alone (i.e., autonomous proliferation) was confirmed (Fig. 3).

All cell cultures were performed in DMEM medium containing 10% fetal bovine serum (FBS) in a 5% CO₂ incubator at 37°C.

2-2.
Adiponectin-expressing Treg cells capable of autonomous proliferation even in an environment lacking thymic stromal cells obtained in Test Example 2-1 (which may be referred to below as "autonomously proliferative adiponectin-expressing Treg cells"), and the Treg cells sorted in Test Example 1-2, were each seeded in DMEM medium containing 10% fetal bovine serum (FBS). After these cells were cultured in the absence of thymic stromal cells in a 5% CO₂ incubator at 37°C for 72 hours, apoptosis was detected using fragmentation of chromatin DNA into nucleosome units (185 bp) as an indicator. Specifically, using an Apoladder Ex kit (Takara Bio Inc.), the presence or absence of DNA ladders was evaluated according to the manufacturer's instructions. Fig. 4a shows the results.

Further, each cell cultured in an environment lacking the thymic stromal cells was mixed with FITC-labeled annexin V and propidium iodide (PI) (PromoCell GmbH), and the cells that underwent apoptosis were measured using the Guava^{®} EasyCyte cell analysis system (Luminex Corporation). The results of the autonomously proliferative adiponectin-expressing Treg cells are shown on the left side of Fig. 4b, whereas the results of the Treg cells sorted in Test Example 1-2 are shown on the right side of Fig. 4b. In four regions in each graph of Fig. 4b, the plots located in the lower-left quadrant represent live cells, the plots located in the lower-right quadrant represent early apoptotic cells, and the plots located in the upper-right quadrant represent late apoptotic cells.

As shown in Figs. 4a and 4b, the results show that autonomously proliferative adiponectin-expressing Treg cells did not undergo apoptosis in an environment lacking thymic stromal cells, whereas the Treg cells sorted in Test Example 1-2 underwent apoptosis in an environment lacking thymic stromal cells.

2-3.
The proliferation rate of autonomously proliferative adiponectin-expressing Treg cells obtained in Test Example 2-1 and the proliferation rate of Treg cells capable of proliferating only in a co-culture system with thymic stromal cells obtained in Test Example 1-1 were compared: the former under monoculture conditions and the latter within a co-culture system. Specifically, the number of Treg cells at each time point was counted using a hemocytometer. The number of autonomously proliferative adiponectin-expressing Treg cells was 3.5 × 10³ (standard deviation: 1.0) at 24 hours after the start of culture and was 10.1 × 10³ (standard deviation: 2.2) at 48 hours, whereas the number of Treg cells capable of proliferating only in a co-culture system with thymic stromal cells was 2.3 × 10³ (standard deviation: 0.8) at 24 hours and was 5.6 × 10³ (standard deviation: 1.4) at 48 hours after the start of culture. Fig. 5 shows the results. In the graph, the ordinate represents the number of Treg cells, and the abscissa represents the time elapsed from the start of culture. The error bars show standard deviations. The dashed line shows the results of autonomously proliferative adiponectin-expressing Treg cells, whereas the solid line represents the results of Treg cells capable of proliferating only in the co-culture system with thymic stromal cells.

As shown in Fig. 5, autonomously proliferative adiponectin-expressing Treg cells were found to have a higher proliferative capacity than Treg cells capable of proliferating only in a co-culture system with thymic stromal cells.

2-4.
Autonomously proliferative adiponectin-expressing Treg cells obtained in Test Example 2-1 and Treg cells capable of proliferating only in the co-culture system with thymic stromal cells obtained in Test Example 1-1 were immunostained with DAPI (blue), fluorescence-labeled rabbit anti-adiponectin antibody (green) (GeneTex), and fluorescence-labeled mouse anti-FOXP3 antibody (red) (Proteintech), as in Test Example 1-3, and observed using a confocal laser scanning microscope (Leica). Fig. 6a shows the results of the autonomously proliferative adiponectin-expressing Treg cells obtained in Test Example 2-1. Fig. 6b shows the results of the Treg cells capable of proliferating only in a co-culture system with thymic stromal cells obtained in Test Example 1-1. Note that in Figs. 6a and 6b, the scale bars represent 20 µm.

As shown in Fig. 6a, it was confirmed that adiponectin was expressed in most of the autonomously proliferative adiponectin-expressing Treg cells obtained in Test Example 2-1. In contrast, as shown in Fig. 6b, adiponectin was expressed only in some of the Treg cells capable of proliferating only in a co-culture system with thymic stromal cells obtained in Test Example 1-1. These results reveal that when Treg cells capable of proliferating only in a co-culture system with thymic stromal cells are applied to the technique of the present disclosure, it is necessary to pre-select cells that express adiponectin, whereas when autonomously proliferative adiponectin-expressing Treg cells are used, such selection is not required.

2-5.
Autonomously proliferative adiponectin-expressing Treg cells obtained in Test Example 2-1, adiponectin-expressing cells selected from Treg cells capable of proliferating only in a co-culture system with thymic stromal cells obtained in Test Example 1-1 (referred to below as "adiponectin-expressing Treg cells selected from the co-culture system"), and thymus-derived cells not expressing adiponectin (control) were subjected to western blotting by the same method as in Test Example 1-4 to detect adiponectin. Fig. 7 shows the results.

In Fig. 7, lane 1 shows the control, lane 2 shows the autonomously proliferative adiponectin-expressing Treg cells obtained in Test Example 2-1, and lane 3 shows the adiponectin-expressing Treg cells selected from the co-culture system. As is clear from the comparison of the band patterns in lanes 2 and 3, uniformly sized high-molecular-weight (HMW) adiponectin was expressed in the autonomously proliferative adiponectin-expressing Treg cells, whereas HMW adiponectin of various molecular weights was expressed in the adiponectin-expressing Treg cells selected from the co-culture system.

### 2-6. Brief Summary

The above test results indicate that gradually decreasing the ratio of thymic stromal cells in the co-culture system of thymic stromal cells and adiponectin-expressing Treg cells enables the acquisition of adiponectin-expressing Treg cells that are resistant to apoptosis (i.e., that are autonomously proliferative) even in an environment lacking thymic stromal cells. It was also found that autonomously proliferative adiponectin-expressing Treg cells exhibit higher proliferation capacity than Treg cells capable of proliferating only in a co-culture system with thymic stromal cells, that when autonomously proliferative adiponectin-expressing Treg cells are used in the technique of the present disclosure, pre-selection of adiponectin-expressing cells from the cell population is not necessary, that the Treg cells uniformly express high-molecular-weight (HMW) adiponectin, and that uniform molecular weight HMW adiponectin is expressed in the autonomously proliferative adiponectin-expressing Treg cells.

### Test Example 3. Investigation of the Effects of Humoral Recombinant Adiponectin on Triple-negative Breast Cancer 3-1

Triple-negative breast cancer cell line MDA-MB-157 (American Type Culture Collection) was cultured in DMEM medium containing high glucose (4500 mg/L) and 10% fetal bovine serum (FBS) in a 5% CO₂ incubator at 37°C. MDA-MB-157 cells were thawed from a liquid nitrogen tank and then subjected to a test within 6 months.

3-2.
Cultured MDA-MB-157 cells were mixed with eukaryotic recombinant human adiponectin (Recombinant Human Adiponectin/Acrp30 Protein, CF, R&D Systems) at concentrations ranging from 0 to 5 µg/mL and incubated for 36 hours. The eukaryotic recombinant human adiponectin was not contained within the cell and was monomeric. In the present disclosure, adiponectin not enclosed within the cells is referred to as "humoral" adiponectin.

The MDA-MB-157 cells after incubation were mixed with FITC-labeled annexin V and propidium iodide (PI) (PromoCell GmbH), and cells that underwent apoptosis were evaluated using a Guava^{®} EasyCyte cell analyzer (Luminex). Fig. 8a shows the results at an adiponectin concentration of 0 µg/mL (control); Fig. 8b shows those at 0.05 ug/mL; Fig. 8c shows those at 0.5 µg/mL; and Fig. 8d shows those at 5 µg/mL. The numerical values prominently shown in each figure indicate the percentage (%) of cells that underwent apoptosis among the evaluated cells.

As shown in Figs. 8a to 8d, the percentage of MDA-MB-157 cells that underwent apoptosis increased in an adiponectin dose-dependent manner. However, even at the highest adiponectin concentration of 5 µg/mL, the percentage of the cells that underwent apoptosis was less than 10%.

These results indicate that humoral adiponectin can induce apoptosis in cancer cells, but with a very low efficiency.

### Test Example 4. Detection of T-cadherin in Cancer Cells

Triple-negative breast cancer cell lines MDA-MB-157 and MDA-MB-231, lobular carcinoma cell line MDA-MB-330 (American Type Culture Collection), and luminal A breast cancer cell line MCF-7 (Japanese Collection of Research Biosources Cell Bank) were cultured in the same manner as in Test Example 3-1. Luminal A breast cancer is breast cancer that is a hormone receptor-positive (estrogen receptor and/or progesterone receptor-positive) HER2-negative breast cancer.

T-cadherin was detected in each cultured cell by western blotting. Specifically, the cell lysate was subjected to electrophoresis using sodium dodecyl sulfate-polyacrylamide gel electrophoresis (SDS-PAGE), and then transferred to a polyvinylidene fluoride membrane (Immobilon-P Transfer Membrane; available from Merck). The membrane was blocked with blocking milk (Megmilk Snow Brand Co., Ltd.) and then incubated with an anti-T-cadherin antibody. For immunodetection, horseradish peroxidase (HRP)-labeled anti-rabbit secondary antibody (Cell Signaling Technology) and ultra-sensitive HRP luminescent substrate (TaKaRa Bio) were used. Images were obtained using the Invitrogen iBright 1500 gel imaging system (Thermo Fisher Scientific). After stripping antibodies from the membrane, the membrane was incubated with an anti-GAPDH antibody (Merck) to evaluate the input. Fig. 9 shows the results. The numerical values shown on the left side of Fig. 9 indicate molecular weight (kDa).

In Fig. 9, lane 1 shows the lobular carcinoma cell line MDA-MB-330, lane 2 shows the triple-negative breast cancer cell line MDA-MB-231, lane 3 shows the triple-negative breast cancer cell line MDA-MB-157, and lane 4 shows the luminal A breast cancer cell line MCF-7. A very faint band of 105 kDa T-cadherin (indicated with an asterisk in Fig. 9) was observed in lane 3, but no T-cadherin bands were seen in lanes 1, 2, or 4. The band near 31 kDa in Fig. 9 shows GAPDH (loading control).

The above results reveal that in the above cancer cell lines, expression of T-cadherin, which is a receptor for both the hexamer and HMW adiponectin, is either barely detectable or detected only in trace amounts.

### Test Example 5. Co-culture of Adiponectin-expressing Treg Cells and Triple-negative Breast Cancer Cells

5-1.
The adiponectin-expressing Treg cells obtained in Test Example 2 were co-cultured with the triple-negative breast cancer cell line MDA-MB-157 or MDA-MB-231 described in Test Example 4. Specifically, the cells were cultured using DMEM medium containing high glucose (4500 mg/L) and 10% fetal bovine serum (FBS) in a 5% CO₂ incubator at 37°C.

5-2.
Figs. 10a to 10d show time-lapse images captured using CytoWatcher (ATTO Inc.) during the co-culture of adiponectin-expressing Treg cells and MDA-MB-157 cells. Fig. 10a shows MDA-MB-157 cells before co-culture. Figs. 10b and 10c show images from different fields of view 16 hours after the start of co-culture. Fig. 10d is an image taken 64 hours after the start of co-culture. In Figs. 10b and 10c, as indicated by the arrows, adiponectin-expressing Treg cells were internalized into the cytoplasm of MDA-MB-157 cells. That is, adiponectin-expressing Treg cells interact with MDA-MB-157 cells to exhibit a cell-in-cell phenomenon. Further, as shown in Fig. 10d, MDA-MB-157 cells that had undergone cell death were observed at 64 hours after the start of co-culture of adiponectin-expressing Treg cells and MDA-MB-157 cells. Note that the arrows in Fig. 10d indicate the cell remnants after cell death.

5-3.
Adiponectin-expressing Treg cells and MDA-MB-157 cells were co-cultured, and cells 16 hours after the start of co-culture were immunostained and photographed with a confocal laser scanning microscope (Leica). Figs. 10e to 10g show the images. For immunostaining, DAPI (blue), fluorescently labeled rabbit anti-adiponectin antibody (green) (GeneTex), and LuminiCell Tracker^{™} 540 (red) (Merck) were used. In Figs. 10e to 10g, blue shows nuclei, green shows adiponectin-expressing Treg cells, and red shows the cytoplasm of MDA-MB-157 cells.

As indicated by the arrowhead in Fig. 10e, adhesion of adiponectin-expressing Treg cells to MDA-MB-157 cells was observed. As indicated by the arrow in Fig. 10e, cytoplasmic fusion between an adiponectin-expressing Treg cell and an MDA-MB-157 cell was observed. Fig. 10f shows a horizontal intracellular image, and Fig. 10g shows a vertical intracellular image of the same subject as Fig. 10f. Immunostaining also confirmed that adiponectin-expressing Treg cells were engulfed by MDA-MB-157 cells.

5-4.
Adiponectin-expressing Treg cells and MDA-MB-231 cells were co-cultured, and the cells 16 hours after the start of co-culture were immunostained. Figs. 10h and 10i show images of the cells taken using a confocal laser scanning microscope (Leica). Immunostaining was performed in the same manner as in Test Example 1-3. Red indicates the cytoplasm of the MDA-MB-231 cells. As indicated by the arrows in Figs. 10h and 10i, it was confirmed that similar to MDA-MB-157 cells, MDA-MB-231 cells also engulfed adiponectin-expressing Treg cells.

### 5-5. Brief Summary

The above results show that adiponectin-expressing Treg cells can interact with cancer cells to exhibit a cell-in-cell phenomenon and can induce cancer cell death.

### Test Example 6. Investigation of Suppressive Effect of Adiponectin-expressing Treg Cells on Peritoneal Mesothelioma

The present inventors investigated whether adiponectin-expressing Treg cells can also exert anticancer effects in vivo.

1 × 10⁶ human mesothelioma MPM-1 cells were intraperitoneally injected into NOD/SCID mice. MPM-1 cells were prepared by the method described in Non-patent Literature (NPL) 6.

Seven days after the injection of MPM-1 cells, adiponectin-expressing Treg cells treated with mitomycin C (10 µg/mL) for 2 hours and 15 minutes and then washed with PBS(-) were suspended in 0.5 mL of PBS and injected intraperitoneally into the mice. The dosage of adiponectin-expressing Treg cells was set at 1×10⁶ cells per mouse. 0.5 mL of PBS was injected intraperitoneally into the mice of the control group. Each group consisted of 5 mice (n=5).

The mitomycin C treatment mentioned above is a treatment for suppressing the proliferation of adiponectin-expressing Treg cells in the mouse body after administration. It is a method commonly used in this technical field. That is, the adiponectin-expressing Treg cells in this Test Example were derived from mice. Therefore, if administered in an untreated state to NOD/SCID mice, the adiponectin-expressing Treg cells may proliferate in the mouse bodies, which potentially affects the test results. This possibility can be eliminated by treating the cells with mitomycin C before administration. After mitomycin C treatment, adiponectin-expressing Treg cells were washed with PBS(-). Therefore, mitomycin C did not remain in the adiponectin-expressing Treg cells that were administered into the mouse body.

Twenty-one days after the injection of MPM-1 cells, each mouse was dissected to observe the proliferation status of intraperitoneal mesothelioma. Fig. 11a shows the results of the control group into which adiponectin-expressing Treg cells were not injected, whereas Fig. 11b shows the results of the group into which adiponectin-expressing Treg cells were injected. The arrows in Fig. 11a and Fig. 11b indicate the intraperitoneal mesothelioma masses.

Further, peritoneal mesotheliomas were excised from each mouse, and their weights were measured. The total weight of intraperitoneal mesothelioma in each group (n = 5) ± standard deviation was calculated from the measurement results. The total weight in the control group was 2.24 ± 0.66 g, whereas that in the group into which adiponectin-expressing Treg cells were injected was 0.38 ± 0.31 g. Further, statistical analysis using Student's t-test indicated P < 0.01.

The above results show, with statistical significance, that adiponectin-expressing Treg cells suppress intraperitoneal mesothelioma growth.

Fig. 11c shows a representative example of the control group. Fig. 11d shows a representative example of the group into which adiponectin-expressing Treg cells were injected. In Fig. 11c, the arrow points to the mesothelioma nodule. Further, Fig. 11e shows a histological diagram of the mesothelioma nodule excised from the control group. After the excised mesothelioma nodule was fixed in formalin and embedded in paraffin, the mesothelioma nodule was sectioned and then stained with hematoxylin and eosin (HE staining). The upper image in Fig. 11e shows an HE-stained specimen of a mesothelioma nodule from the control group, while the lower image presents an enlarged view of the same specimen. As shown in Fig. 11e, vigorous invasive proliferation into the surrounding striated muscle tissue around the mesothelioma nodule was observed. Mesothelioma nodules demonstrating such marked proliferative ability were observed in the control group as shown in Figs. 11a and 11c, whereas such mesothelioma nodules were barely observed in the group into which adiponectin-expressing Treg cells were injected as shown in Figs. 11b and 11d.

As shown in Fig. 11e, mesothelioma cells proliferated in the mesothelioma nodules of the control group. The results shown in Figs. 11c to 11e revealed that mesothelioma nodule formation was significantly suppressed in the group into which adiponectin-expressing Treg cells were injected.

The above results show that adiponectin-expressing Treg cells can exert anti-tumor effects in vivo as well.

## Claims

1. A composition for preventing and/or ameliorating cancer, the composition comprising regulatory T cells that express adiponectin.

2. The composition according to claim 1, wherein the regulatory T cells interact with cancer cells to exhibit a cell-in-cell phenomenon.

3. The composition according to claim 1, wherein the regulatory T cells express HMW adiponectin.

4. The composition according to claim 2, wherein the cancer has a low adiponectin receptor expression level.

5. The composition according to claim 4, wherein the adiponectin receptor is T-cadherin.

6. The composition according to any one of claims 1 to 5, wherein the cancer is at least one selected from the group consisting of breast cancer, mesothelioma, bladder cancer, and peritoneally disseminated cancer.

7. The composition according to any one of claims 1 to 5, wherein the cancer is breast cancer and/or mesothelioma.

8. The composition according to any one of claims 1 to 5, wherein the cancer is triple-negative breast cancer.

9. The composition according to any one of claims 1 to 5, which is a pharmaceutical composition.

10. A method for producing regulatory T cells that are capable of proliferating in a culture system in the absence of thymic stromal cells and that express adiponectin,
the method comprising the steps of
(A) co-culturing regulatory T cells that express adiponectin with thymic stromal cells; and
(B) co-culturing the regulatory T cells that express adiponectin with the thymic stromal cells under conditions in which the ratio of the thymic stromal cells to the regulatory T cells at the start of co-culture is lower than that at the start of the previous co-culture.
